# EUROPÄISCHE PATENTSCHRIFT

(11) **EP 0 605 504 B1**
(45) Veröffentlichungstag und Bekanntmachung des Hinweises auf die Patenterteilung: **19.06.1996**
(21) Anmeldenummer: 92919597.2
(22) Anmeldetag: 17.09.1992
(51) Int. Cl.: A61K 7/00, A61K 7/48, A61K 31/20

(54) **KOSMETISCHE UND DERMATOLOGISCHE ZUBEREITUNGEN**
COSMETIC AND DERMATOLOGICAL PREPARATIONS
PREPARATIONS COSMETIQUES ET DERMATOLOGIQUES

(30) Priorität: 25.09.1991 DE 4131940
(43) Veröffentlichungstag der Anmeldung: 13.07.1994
(73) Patentinhaber: Beiersdorf Aktiengesellschaft, D-20245 Hamburg (DE)
(72) Erfinder: HERPENS, Andreas, D-2057 Reinbek (DE); JACOB, Jürgen, D-2000 Hamburg 65 (DE); SAUERMANN, Gerhard, D-2351 Wiemersdorf (DE); SCHOTTEN, Theo, D-2000 Hamburg 20 (DE); SCHREINER, Volker, D-2000 Hamburg 20 (DE)
(86) Internationale Anmeldenummer: DE9200797
(87) Internationale Veröffentlichungsnummer: WO9305752

(56) Entgegenhaltungen:
- Chemical Abstracts, Band 109, no. 18, 31 Oktober 1988, (Columbus, Ohio, US), siehe Seite 422, Zusammenfassung 155965m, & CS, , 238470 (Cosmetics, pharmaceuticals or/and detergents based on lipid component) 1987
- Chemical Abstracts, Band 105, no. 20, 17 November 1986, (Columbus, Ohio, US), siehe Seite 363, Zusammenfassung 178231a, & CS,, 229971 (Cosmetic, pharmaceutical and/or surfactant compositions containing animal lipid component) 1986
- Chemical Abstracts, Band 83, no. 24, 15 Dezember 1975, (Columbus, Ohio, US), siehe Seite 284, Zusammenfassung 197697a, & JP, , 7511340 ((SANSEI DRUG MFG. CO., LTD.)) 1975
- Chemical Abstracts, Band 94, no. 4, 26 Januar 1981, (Columbus, Ohio, US), siehe Seite 298, Zusammenfassung 20249g, & JP,, 80115812 (Oil/water-type cosmetic emulsions) 1980
- Chemical Abstracts, Band 108, no. 10, 7 März 1988, (Columbus, Ohio, US), siehe Seite 425, Zusammenfassung 81841y, & JP,, 62238210 ((POLA CHEMICAL INDUSTRIES, INC.)) 1987
- Patent Abstracts of Japan, Band 8, Nr 11, C205, Zusammenfassung von JP 58- 177908, publ 1983-10-18

## Beschreibung

Die vorliegende Erfindung betrifft insbesondere Zubereitungen zur Pflege trockener und beanspruchter Haut und Altershaut sowie zur Behandlung und Prophylaxe von Folgeschäden der Hautaustrocknung, beispielsweise Fissuren und inflammatorische oder allergische Prozesse.

Die Behandlung der Haut mit waschaktiven Substanzen bewirkt im Nebeneffekt die Elution von Epidermallipiden (Sebum, Interzellularlipide) und hygroskopischen Stoffen von der Oberfläche der Hornschicht. Zwischen natürlicher Regeneration dieser essentiellen Substanzen und ihrem ständigen Verlust durch regelmäßige Extraktion stellt sich ein Gleichgewichtszustand ein, der äußeres Erscheinungsbild der Haut und physiologische Funktion der Hornschicht entscheidend mit beeinflußt.

Bei alter Haut erfolgt der regenerative Turnover verlangsamt, wobei insbesondere das Wasserbindungsvermögen der Hornschicht nachläßt. Sie wird deshalb unflexibel, trocken und rissig ("physiologisch" trockene Haut). Ein Barriereschaden ist die Folge. Die Haut wird anfällig für negative Umwelteinflüsse wie die Invasion von Mikroorganismen, Toxinen und Allergenen. Bei pathologisch trockener und empfindlicher Haut liegt ein Barriereschaden a priori vor. Epidermale Interzellularlipide werden offenbar fehlerhaft oder in ungenügender Menge bzw. Zusammensetzung gebildet. Die Konsequenz ist eine erhöhte Durchlässigkeit der Hornschicht und ein unzureichender Schutz der Haut vor Verlust an hygroskopischen Substanzen und Wasser.

Zubereitungen zur Pflege und Behandlung trockener Haut sind an sich bekannt. Ihr Beitrag zur Regeneration einer physiologisch intakten, hydratisierten und glatten Hornschicht ist allerdings umfangmäßig und zeitlich begrenzt.

Die Wirkung von bekannten Salben und Cremes auf Barrierefunktion und Hydratation der Hornschicht besteht nur teilweise in einer Wiederherstellung beziehungsweise Stärkung der physikalisch-chemischen Eigenschaften der Lamellen aus Interzellularlipiden. Ein wesentlicher Teileffekt beruht auf der bloßen Abdeckung der behandelten Hautbezirke und dem daraus resultierenden Wasserstau in der darunterliegenden Hornschicht. Entsprechend leicht kann diese physikalische Barriere wieder entfernt werden. Darüberhinaus kann die Hautpflegewirkung bei regelmäßiger Behandlung nachlassen, so daß schließlich sogar der Status quo wieder erreicht wird. Nach dem Absetzen der Produktanwendung kehrt die Haut sehr schnell wieder in den Zustand vor Behandlungsbeginn zurück. Bei bestimmten Produkten verschlechtert sich der Zustand der Haut unter Umständen vorübergehend. Eine nachhaltige Wirkung wird in der Regel also nicht oder nur in einem eingeschränkten Maße erreicht.

Aufgabe der vorliegenden Erfindung ist es also, die Nachteile des Standes der Technik zu beseitigen. Insbesondere sollen stark wirkende kosmetische und dermatologische Zubereitungen geschaffen werden, die dazu dauerhaft und nachhaltig die beanspruchte Haut, insbesondere die pathologisch trockene Haut und die Altershaut, pflegen und glätten. Der erzielte Effekt soll auch nach dem Ende der Anwendung noch eine gewisse Zeit erhalten bleiben. Sie sollen ferner zur Behandlung und Prophylaxe von Folgeschäden der Hautaustrocknung, beispielsweise Fissuren oder inflammatorischen oder allergischen Prozessen oder auch der Neurodermitis geeignet sein.

Diese Aufgabe wird gelöst durch kosmetische und dermatologische Zubereitungen, die mindestens ein Neutrallipid und mindestens eine mono-cis-Alkencarbonsäure oder deren Salz enthalten, wobei der Anteil der mono-cis-Alkencarbonsäure 0,05 bis 0,5 Gewichtsprozent bezogen auf das Gesamtgewicht der Zubereitung beträgt.

Bevorzugt werden topische, kosmetische oder dermatologische Zubereitungen.

Es hat sich überraschenderweise gezeigt, daß die erfindungsgemäßen Zubereitungen mit mono-cis-Alkencarbonsäuren oder ihren Salzen in dem niedrigen, eng gefaßten Konzentrationsbereich in signifikanter Weise den Nachteilen des Standes der Technik abhelfen. So wirken Neutrallipid und mono-cis-Alkencarbonsäure synergistisch zusammen, wie auch der Versuchsbericht zeigt.

Überraschenderweise bleibt die erfindungsgemäße, z.B. pflegende und glättende Wirkung der erfindungsgemäßen Kombinationen auch noch lange Zeit nach dem Ende der Behandlung erhalten.

Es können auch Gemische der Neutrallipide und/oder der mono-cis-Alkencarbonsäuren, beispielsweise mit jeweils 2, 3, 4, 5 oder 6 Komponenten eingesetzt werden. Insbesondere Ceramide können als Gemische verwendet werden.

Geeignete Neutrallipide sind insbesondere Wollwachsalkohole, Cholesterin und Cholesterinderivate, beispielsweise Cholesterincarbonsäureester, Ceramid, Squalen, Wachsester oder Triglyceride und Gemische dieser Lipide. Sie sind bekannt und auch im Handel erhältlich.

Bevorzugte Neutrallipide sind weiterhin Wollwachsalkohole, Cholesterin, Cholesterincarbonsäureester, Ceramide oder Squalen und Gemische dieser Lipide.

Die Neutrallipide können beispielsweise in einer Menge von 0,05 bis 95 Gewichts-%, bezogen auf das Gesamtgewicht, in den Zubereitungen enthalten sein.

Bevorzugt werden solche Neutrallipid-Gemische die Cholesterin oder Cholesterinderivate oder Wollwachsalkohole oder Ceramid oder ein Ceramidgemisch oder auch Gemische aus solchen ausgewählten Lipiden enthalten.

Besonders bevorzugt sind erfindungsgemäße Zubereitungen, die Cholesterin, Cholesterinderivate oder Wollwachsalkohole und/oder Ceramid enthalten.

Geeignete Triglyceride sind z.B. Verbindungen, in denen Glycerin mit drei organischen, gegebenenfalls unterschiedlichen Säureestern verestert ist. Sie werden bevorzugt gewählt aus der Gruppe der Glycerylcaproate, -caprylate, -linoleate, -stearate und/oder -palmitate.

Das Cholesterin und/oder Wollwachsalkohole können aus gereinigtem Wollfett des Schafes erhalten werden. Als Wollwachsalkohole können Handelsprodukte wie z.B. Eucerit^{R} oder Eucerinum Anhydricum^{R} (Beiersdorf Aktiengesellschaft) verwendet werden. Es hat sich als besonders vorteilhaft erwiesen, das Cholesterin in Lösungsmitteln, besonders bevorzugt ist Cetyloctanoat, zu lösen und den Formulierungen als Lösung zuzugeben.

Squalen kann vorteilhaft aus den aus Fischleberöl, insbesondere Haifischleberöl, gewonnenen Produkten gewählt werden.

Als Wachsester hat sich das Jojobaöl als besonders vorteilhaft herausgestellt.

Ceramide können aus natürlichen Vorkommen isoliert oder in naturidentischer Form synthetisiert werden. Bevorzugt werden Spingosylstearat, Spingomyelin (Firma Sigma, USA) und Spingoceryl.

Die erfindungsgemäßen mono-cis-Alkencarbonsäuren sind insbesondere ungesättigte aliphatische Monocarbonsäuren mit einer C=C-Doppelbindung, an der sich zwei Alkylreste bzw. Kohlenstoffatome in cis-Position befinden.

Vorzugsweise liegt die Doppelbindung im Bereich der Molekülmitte oder in der Molekülmitte.

Bevorzugte mono-cis-Alkencarbonsäuren sind geradkettige oder verzweigte Carbonsäuren mit vorzugsweise 12 - 24 Kohlenstoffatomen und einer cis-Doppelbindung. Bevorzugt werden unverzweigte Carbonsäuren oder gering verzweigte, insbesondere solche mit kurzen Seitenketten wie Methylgruppen oder Ethylgruppen, beispielsweise ein oder zwei Methyl- und/oder Ethylgruppen. Es können auch Gemische der erfindungsgemäßen mono-cis-Alkencarbonsäuren eingesetzt werden.

Besonders bevorzugt sind geradkettige, unverzweigte mono-cis-Alkencarbonsäuren mit 16 - 20 Kohlenstoffatomen und deren Salze.

Vorzugsweise können die folgenden erfindungsgemäßen Alkencarbonsäuren verwendet werden:

Ölsäure, Palmitoleinsäure, cis-6-Hexadecensäure. Besonders bevorzugt wird Ölsäure.

Gut geeignete Salze der erfindungsgemäßen Alkencarbonsäuren sind wasserlösliche Salze, beispielsweise Alkalisalze wie die Natrium- und Kalium-Salze oder Ammoniumsalze.

Die erfindungsgemäßen Alkencarbonsäuren sind bekannt, einige sind auch im Handel erhältich. Sie können auch nach bekannten Verfahren erhalten werden.

Zweckmäßigerweise wird der pH-Wert der Zubereitungen für Hautbedingungen passend eingestellt, z.B. in den Bereichen pH 5 bis pH 7,5, bevorzugt pH 5,5, bis pH 6.

Die erfindungsgemäßen kosmetischen und dermatologischen Zubereitungen können als topische Zubereitungen, als Hautpflegeprodukte und Dermatika insbesondere zur Pflege trockener, empfindlicher oder beanspruchter Haut, Altershaut und pathologisch trockener Haut sowie zur Behandlung und Prophylaxe von Folgeschäden der Hautaustrocknung, beispielsweise Fissuren oder inflammatorischen oder allergischen Prozessen verwendet werden.

Sie können auch zur Behandlung und Prophylaxe der Neurodermitis dienen. Hierzu werden die Zubereitungen einmal oder mehrmals täglich auf erkrankte Hautstellen aufgetragen.

Gegenstand der Erfindung ist daher auch die kosmetische Verwendung der Zubereitungen als Hautpflegeprodukte, zur Pflege trockener Haut und von Altershaut, sowie die Verwendung der erfindungsgemäßen Kombination aus Neutrallipid und mono-cis-Alkencarbonsäure zur Herstellung einer kosmetischen oder dermatologischen Zubereitung, insbesondere zur Behandlung der Neurodermitis und der pathologisch trockenen Haut.

Vorzugsweise enthalten die erfindungsgemäßen kosmetischen und dermatologischen Zubereitungen ein Neutrallipid oder mehrere Neutrallipide in einer Menge von 0,5 bis 20 oder 0,5 bis 10 Gewichts-%, insbesondere 1 bis 5 Gewichts-% bezogen auf das Gesamtgewicht der Zubereitung.

Eine oder mehrere erfindungsgemäße mono-cis-Alkencarbonsäuren sind vorzugsweise in Mengen von 0,2 bis 0,4 Gewichts-%, insbesondere aber 0,3 Gewichts-%, bezogen auf das Gesamtgewicht in den Zubereitungen enthalten.

Dabei ist die Gewichtsmenge der Neutrallipide vorzugsweise gleich der Gewichtsmenge der mono-cis-Alkencarbonsäuren oder größer, und sie beträgt insbesondere das 1 bis 10-fache oder 1 bis 25-fache dieser Menge. Bevorzugt beträgt die Gewichtsmenge der Lipide das 5 bis 15-fache, insbesondere das 7 bis 12-fache oder etwa das 10-fache des Alkencarbonsäuregewichtes.

Bevorzugt werden weiterhin Kombinationen von Neutrallipiden und Carbonsäuren in den folgenden Gewichts-%-Mengen bezogen auf das Gesamtgewicht der Zubereitungen:
- 0,05 bis 6 Gewichts-%: Neutrallipid und
- 0,05 bis 0,5 Gewichts-%: mono-cis-Alkencarbonsäure
oder
- 1 bis 5 Gewichts-%: Neutrallipid und
- 0,2 bis 0,4 Gewichts-%: mono-cis-Alkencarbonsäure
oder
- 3 Gewichts-%: Neutrallipid und
- 0,3 Gewichts-%: mono-cis-Alkencarbonsäure,
und besonders bevorzugt werden
- 0,05 bis 6 Gewichts-%: Cholesterin, Cholesterinderivate oder Wollwachsalkohole und/oder Ceramid und
- 0,05 bis 0,5 Gewichts-%: Ölsäure
oder
- 1 bis 5 Gewichts-%: Cholesterin, Cholesterinderivate oder Wollwachsalkohole und/oder Ceramid und
- 0,2 bis 0,4 Gewichts-%: Ölsäure
oder
- 3 Gewichts-%: Cholesterin, Cholesterinderivate oder Wollwachsalkohole und/oder Ceramid und
- 0,3 Gewichts-%: Ölsäure.

Im Rahmen der vorliegenden Anmeldung sind, soweit nicht anders angegeben, alle Mengen und Prozentangaben auf das Gewicht und die Gesamtzusammensetzung der Zubereitung bezogen.

Die erfindungsgemäßen Neutrallipide und mono-cis-Alkensäuren können in allen bekannten kosmetischen und dermatologischen Zubereitungen enthalten sein oder in diese eingearbeitet werden. Es sind dies insbesondere Emulsionen, Cremes, Lotionen, Milchen, Salben, Fettsalben, Gele, Solubilisate, Lösungen, Suspensionen, ölige Zubereitungen oder feste, beispielsweise stiftförmige Zubereitungen oder Sprays in allen Formen.

Wenn sich auch die erfindungsgemäßen Zusammensetzungen allen topischen, also kosmetischen sowie dermatologischen Zubereitungen zufügen lassen, so werden doch W/O-Emulsionen und O/W-Emulsionen und Öle bevorzugt. Vorteilhaft können die erfindungsgemäßen Neutrallipid-CarbonsäureKombinationen in O/W-Cremes, W/O-Cremes, O/W-Lotionen und W/O-Lotionen eingesetzt werden.

Die erfindungsgemäßen Neutrallipid-Alkensäure-Kombinationen können auch vorteilhaft als solche eingesetzt werden und somit kosmetische und dermatologische Zubereitungen darstellen. Es ist aber vorteilhafter, diese Kombinationen in übliche kosmetische und dermatologische Grundlagen einzuarbeiten. Dabei sind die üblichen Regeln der Fertigungspraxis zu beachten, welche dem Fachmanne geläufig sind. Ferner weiß der Fachmann, daß solchen Produkten übliche Hilfs- und/oder Zusatzstoffe zugefügt werden können, ohne daß der Rahmen der vorliegenden Erfindung dadurch verlassen würde.

So ist es von Vorteil, den Zubereitungen Antioxidantien, z.B. alpha-Tocopherol, zuzusetzen, um die Stabilität der oxidationsempfindlichen cis-Alkencarbonsäuren zu gewährleisten. Weitere Hilfsstoffe oder Zusatzstoffe können wasserbindende Stoffe, Verdicker, Füllstoffe, Parfüm, Farbstoffe, Emulgatoren, oder Wirkstoffe wie Desodorantien oder Antitranspirantien, Lichtschutzmittel, Sonnenschutzmittel, UVA-Blocker, UVB-Blocker, Vitamine, Proteine, Insektenrepellentien, Stabilisatoren, Konservierungsmittel, Alkohole, keratolytisch oder proteolytisch wirksame Substanzen, Wasser und/oder Salze, beispielsweise Puffersalze oder Puffergemische zur pH-Einstellung, insbesondere für topische Zwecke sein, z.B. wie sie für Hauteigenschaften und Hautbedingungen bekannt oder erforderlich sind.

Vorzugsweise dienen die erfindungsgemäßen Neutrallipid-Alkensäure-Kombinationen sowie die damit erhaltenen kosmetischen und dermatologischen Zubereitungen zur Behandlung der Haut.

Die Herstellung der erfindungsgemäßen Zubereitungen kann in an sich bekannter Weise erfolgen und ist auch Gegenstand der Erfindung. Vorteilhafterweise wird die erfindungsgemäße Neutrallipid-Carbonsäure-Kombination in der Fettphase der Zubereitungen gelöst, zugemischt oder aber direkt eingearbeitet. Dabei sind die üblichen Maßregeln zu beachten, die dem Fachmann bekannt sind.

Gegenstand der Erfindung ist auch die Kombination eines erfindungsgemäßen Neutrallipids mit einer erfindungsgemäßen mono-cis-Alkencarbonsäure oder jeweils deren Gemischen.

Die folgenden Beispiele dienen zur Erläuterung der Erfindung.

Durch Mischen der angegebenen Bestandteile werden die folgenden erfindungsgemäßen Lipidkombinationen der Beispiele A - F hergestellt.

| Lipidkombination/Beispiel | A | B | C | D | E | F |
|---|---|---|---|---|---|---|
| Neutrallipid | Gewichtsprozent | | | | | |
| Wollwachsalkohole (Eucerit^{R}) | 91 | 99 | 95 | -- | -- | -- |
| Cholesterin | -- | -- | -- | 32 | 16 | 32,3 |
| Sphingosylstearat | -- | -- | -- | 57 | 29 | -- |
| Sphingomyelin (Firma Sigma, USA) | -- | -- | -- | -- | -- | 62 |
| Squalen | -- | -- | -- | -- | 18 | -- |
| Jojobaöl | -- | -- | -- | -- | 3 | -- |
| Glyceryltricaprylat | -- | -- | -- | -- | 25 | -- |
| mono-cis-Alkencarbonsäure cis-6-Hexadecensäure | | | | | | |
| | 9 | -- | -- | -- | -- | -- |
| Ölsäure | - | 1 | 5 | 11 | 9 | 5,7 |

Diese Kombinationen werden den üblichen kosmetischen und dermatologischen Zubereitungen zugefügt. Dabei erweist es sich als günstig, 2,2 Teile der erfindungsgemäßen Zusammensetzung von A, 5,3 Teile von B oder F, 6,3 Teile von C, 2,6 Teile von D oder 5,6 Teile von E zu 93,7 - 97,8 Teilen der jeweiligen Zubereitung zu einer erfindungsgemäßen Formulierung zu vereinen.

Vorteilhaft können die erfindungsgemäßen Lipidkombinationen in Pflegeprodukte wie beispielsweise O/W-Cremes, W/O-Cremes, O/W-Lotionen, W/O-Lotionen usw. eingesetzt werden.

### Beispiel I

### Hautcreme vom W/O-Typ

| Fettphase: | Gew.-% |
|---|---|
| Vaseline DAB 9 | 13 |
| Paraffinöl (Mineralöl 5E,Shell) | 31 |
| Cetearylalkohol/PEG-40-Castor Oil/Natriumcetearylsulfat (Emulgade F, Henkel KGaA) | 2,5 |

| Wasserphase: | |
|---|---|
| Glycerin DAB 9 | 6,3 |
| Wasser, entmineralisiert | 34,4 |

In die 75°C warme Fettphase wird ein Gemisch aus 6,3 Gew.-% der Lipidkombination gemäß Beispiel C und 6,5 Gew.-% Paraffinöl gelöst. Die Fettphase wird sodann zu der 75°C warmen Wasserphase gegeben, verrührt und homogenisiert, bis eine gleichmäßige weiße Creme entstanden ist.

Beispiel I hat folgende endgültige Zusammensetzung:

| | Gew.-% |
|---|---|
| Vaseline DAB 9 | 13 |
| Glycerin DAB 9 | 6,3 |
| Wasser, entmineralisiert | 34,4 |
| Paraffinöl (Mineralöl 5E,Shell) | 37,5 |
| Cetearylalkohol/PEG-40-Castor Oil/Natriumcetearylsulfat (Emulgade F, Henkel KGaA) | 2,5 |
| Wollwachsalkohole (Eucerit^{R}) | 6 |
| Ölsäure | 0,3 |

### Beispiel II

### Hautcreme vom W/O-Typ

| Fettphase: | Gew.-% |
|---|---|
| POE-Glycerolsorbitanoleostearat | |
| (Arlacel 581, ICI) | 8 |
| Vaseline DAB | 2,8 |
| Paraffinwachs/Paraffin | 1,8 |
| Ceresin | 2,2 |
| Octyldodecanol | |
| (Eutanol G, Henkel) | 10 |
| Summe Additive (Parfüm, | |
| Konservierung, Stabilisation) | 0,8 |

| Wasserphase: | |
|---|---|
| Propylenglycol | 1 |
| Glycerin | 1 |
| Magnesiumsulfat | 0,7 |
| Wasser, entmineralisiert | 59,65 |

In die 75°C warme Fettphase wird ein Gemisch aus 5,3 Gew.% der Lipidkombination gemäß Beispiel B und 7 Gew.-% Paraffinöl gelöst. Die Fettphase wird sodann zu der 75°C warmen Wasserphase gegeben, verrührt und homogenisiert, bis eine gleichmäßige weiße Creme entstanden ist.

Beispiel II hat folgende endgültige Zusammensetzung:

| | Gew.-% |
|---|---|
| POE-Glycerolsorbitanoleostearat (Arlacel 581, ICI) | |
| Paraffinwachs | 8 |
| Vaseline DAB | 2,8 |
| Paraffinwachs/Paraffin | 1,8 |
| Paraffinöl (Mineralöl 5E,Shell) | 7 |
| Ceresin | 2,2 |
| Octyldodecanol (Eutanol G, Henkel) | 10 |
| Wollwachsalkohole (Eucerit^{R}) | 5 |
| Ölsäure | 0,05 |
| Propylenglycol | 1 |
| Glycerin | 1 |
| Magnesiumsulfat | 0,7 |
| Wasser, entmineralisiert | 59,65 |
| Summe Additive (Parfüm, Konservierung, Stabilisation) | 0,8 |

### Beispiel III

### Hautcreme vom O/W-Typ

| Fettphase: | Gew.-% |
|---|---|
| Octyldodecanol (Eutanol G, Henkel KGaA) | 9,3 |
| Cetearylalkohol/PEG-40-Castor Oil/Natriumcetearylsulfat (Emulgade F, Henkel KGaA) | 3,7 |
| Paraffinöl (Mineralöl eE, Shell) | 1,4 |

| Wasserphase: | |
|---|---|
| Wasser, entmineralisiert | 73,7 |
| Glycerin DAB 9 | 4,6 |

In die 75°C warme Fettphase wird ein Gemisch aus 2,6 Gew.-% der Lipidkombination gemäß Beispiel D und 4,7 Gew.-% Paraffinöl gelöst. Die Fettphase wird sodann zu der 75°C warmen Wasserphase gegeben, verrührt und homogenisiert, bis eine gleichmäßige weiße Creme entstanden ist.

Beispiel III hat folgende endgültige Zusammensetzung:

| | Gew.-% |
|---|---|
| Octyldodecanol (Eutanol G, Henkel KGaA) | 9,3 |
| Cetearylalkohol/PEG-40-Castor Oil/Natriumcetearylsulfat (Emulgade F, Henkel KGaA) | 3,7 |
| Wasser, entmineralisiert | 73,7 |
| Glycerin DAB 9 | 4,6 |
| Paraffinöl (Mineralöl eE, Shell) | 6,1 |
| Sphingosylstearat | 1,5 |
| Cholesterin | 0,8 |
| Ölsäure | 0,3 |

### Beispiel IV

### O/W-Lotion

| Fettphase: | Gew.-% |
|---|---|
| Steareth-2 | 3 |
| Steareth-21 | 2 |
| Cetearylalkohol/PEG-40-Castor Oil/Natriumcetearylsulfat (Emulgade F, Henkel KGaA) | 2,5 |
| Paraffinöl (Mineralöl eE, Shell) | 3 |

| Wasserphase: | |
|---|---|
| Propylenglycol | 1 |
| Glycerin | 1 |
| Wasser, entmineralisiert | 74,3 |
| Summe Additive (Parfüm, Konservierung, Stabilisation) | 0,8 |

In die 75°C warme Fettphase wird ein Gemisch aus 5,3 Gew.-% der Lipidkombination gemäß Beispiel F und 7 Gew.-% Paraffinöl gelöst. Die Fettphase wird sodann zu der 75°C warmen Wasserphase gegeben, verrührt und homogenisiert, bis eine gleichmäßige weiße Lotion entstanden ist.

Beispiel IV hat folgende endgültige Zusammensetzung:

| | Gew.-% |
|---|---|
| Steareth-2 | 3 |
| Steareth-21 | 2 |
| Cetearylalkohol/PEG-40-Castor Oil/Natriumcetearylsulfat (Emulgade F, Henkel KGaA) | 2,5 |
| Paraffinöl (Mineralöl eE, Shell) | 10 |
| Propylenglycol | 1 |
| Sphingomyelin (Sigma) | 1,6 |
| Cholesterin | 3,5 |
| Ölsäure | 0,3 |
| Glycerin | 1 |
| Wasser, entmineralisiert | 74,3 |
| Summe Additive (Parfüm, Konservierung, Stabilisation) | 0,8 |

### Beispiel V

### O/W-Lotion

| Fettphase: | Gew.-% |
|---|---|
| Octyldodecanol (Eutanol G, Henkel KGaA) | 5,6 |
| Cetearylalkohol/PEG-40-Castor Oil/Natriumcetearylsulfat (Emulgade F, Henkel KGaA) | 8,9 |
| Cetearylisononanoat (Cetiol 5N, Henkel KGaA) | 7,5 |

| Wasserphase: | |
|---|---|
| Wasser, entmineralisiert | 66,1 |
| Glycerin DAB 9 | 4,7 |

In die 75°C warme Fettphase wird ein Gemisch aus 2,2 Gew.-% der Lipidkombination gemäß Beispiel A und 5 Gew.-% Paraffinöl gelöst. Die Fettphase wird sodann zu der 75°C warmen Wasserphase gegeben, verrührt und homogenisiert, bis eine gleichmäßige weiße Lotion entstanden ist.

Beispiel V hat folgende endgültige Zusammensetzung:

| | Gew.-% |
|---|---|
| Octyldodecanol (Eutanol G, Henkel KGaA) | 5,6 |
| Cetearylalkohol/PEG-40-Castor Oil/Natriumcetearylsulfat (Emulgade F, Henkel KGaA) | 8,9 |
| Cetearylisononanoat (Cetiol 5N, Henkel KGaA) | 7,5 |
| Wasser, entmineralisiert | 66,1 |
| Glycerin DAB 9 | 4,7 |
| Paraffinöl (Mineralöl eE, Shell) | 5 |
| Wollwachsalkohole (Eucerit^{R}) | 2 |
| cis-6-Hexadecensäure | 0,2 |

### Beispiel VI

| Hautöl | Gew.-% |
|---|---|
| Glyceryltricaprylat (Miglyol 812, Dynamit Nobel) | 19,4 |
| Hexyllaurat (Cetiol A, Henkel KGaA) | 20 |
| Octylstearat (Cetiol 886, Henkel KGaA) | 20 |
| Paraffinöl (Mineralöl 5E, Shell) | 35 |
| Lipidkombination Beispiel E | 5,6 |

Die Komponenten werden bei 25°C verrührt, bis eine gleichmäßige, klare Mischung entstanden ist.

### Beispiel VI hat folgende endgültige Zusammensetzung:

| | Gew.-% |
|---|---|
| Glyceryltricaprylat (Miglyol 812, Dynamit Nobel) | 20,8 |
| Hexyllaurat (Cetiol A, Henkel KGaA) | 20 |
| Octylstearat (Cetiol 886, Henkel KGaA) | 20 |
| Paraffinöl (Mineralöl 5E, Shell) | 35 |
| Sphingosylstearat | 1,6 |
| Cholesterin | 0,9 |
| Squalen | 1 |
| Jojobaöl | 0,2 |
| Ölsäure | 0,5 |

Die Formulierungen entsprechend den Beispielen sind hochwertige kosmetische und dermatologische Produkte. Nach regelmäßiger Anwendung ist z.B. die Haut im Vergleich zu herkömmlichen Formulierungen (etwa normalen W/O- oder O/W-Cremes) geglättet. Der Effekt hält nach Beendigung der Anwendung noch an.

Für die Prophylaxe und Behandlung der Neurodermitis und der pathologisch trockenen Haut sind die Zubereitungen mit D und F, insbesondere auch die der Beispiele 2, 3 und 4 bevorzugt.

### Versuchsbericht

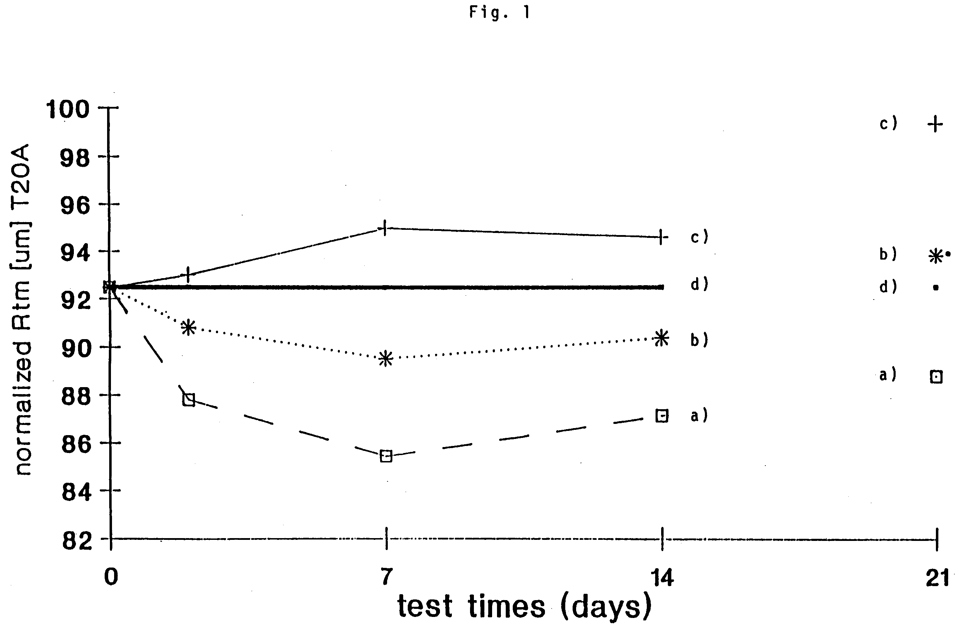

Fig. 1 veranschaulicht die Abnahme der Rauhigkeit der pathologisch trockenen Haut der Arminnenseiten von 7 Probandinnen (mittleres Alter 40 Jahre, S.D. = 9,7) unter Einwirkung einer O/W-Hautcreme (Grundlagenrezeptur s.u.) a) mit 2,6 Gew.-% einer Lipidkombination gemäß Beispiel D, b) mit Neutrallipid gemäß Beispiel D ohne Ölsäure, c) mit 0,3 Gew.-% Ölsäure ohne Neutrallipid (2,3 Gew.-% durch Wasser ersetzt). Die Produkte wurden über zwei Wochen zweimal täglich angewendet. Die Anwendung erfolgte an drei Arealen der Unterarminnenseiten. Als Kontrolle diente ein unbehandeltes Areal (Ergebnisse d). Die Areale wurden über beide Arme durchpermutiert. Dabei erfolgte die Untersuchung der Hautrauhigkeit 12 Stunden nach letztmaliger Produktanwendung. Nach Beendigung der zweiten Anwendungswoche wurde die Applikation gestoppt. Eine Woche später (4. Woche) wurde die Hautrauhigkeit nochmals vermessen. Die Messung der Hautrauhigkeit erfolgte nach der von Hoppe (J. Soc. Cosmet. Chem. 30: 213, 1979) entwickelten Methode. Zur Auswertung gelangt die mittlere Rauhtiefe R_{z}Din (bzw. Rtm, vergl. DIN-Vorschrift 4768, Blatt 1). Die Daten wurden auf die Änderung von R_{z}Din des unbehandelten Kontrollareals normiert.

Beim Produktvergleich wird deutlich erkennbar, daß Ölsäure und Hautlipid synergistisch wirken. Die Hautrauhigkeit nimmt bei Behandlung mit Produkt a) nach einer Woche signifikant gegenüber unbehandelt und Ausgangswert ab (p < 0,05). Der Unterschied zum ölsäurefreien Präparat b) ist nach zwei Anwendungswochen ebenfalls signifikant (p < 0,2). Eine Woche nach Anwendungsstop glättet das Produkt a) signifikant besser als Produkt c) (p < 0,01) bzw. Produkt b) (p < 0,2).

### Zusammensetzung

| | Gew.-% |
|---|---|
| Testsubstanz | 2,6 |
| Trilaureth-4-phosphat (Dehydol LF 3, Henkel) | 2 |
| Sorbitanstearat | 4 |
| Isopropylpalmitat | 4 |
| Cetylstearylalkohol | 7,5 |
| Polyethylenglykol (Polydiol 300) | 3 |
| Sorbit (Karion F, flüssig, Merck) | 5 |
| Wasser, entmineralisiert | 71,9 |

## Patentansprüche

1. Kosmetische und dermatologische Zubereitung, enthaltend mindestens ein Neutrallipid und mindestens eine mono-cis-Alkencarbonsäure oder deren Salz, wobei der Anteil der mono-cis-Alkencarbonsäure 0,05 bis 0,5 Gewichtsprozent bezogen auf das Gesamtgewicht der Zubereitung beträgt.

2. Zubereitung gemäß Anspruch 1, dadurch gekennzeichnet, daß sie als Neutrallipide Wollwachsalkohole, Cholesterin, Cholesterinderivate, Ceramid, Squalen, Wachsester oder Triglyceride enthält.

3. Zubereitung gemäß Anspruch 1, dadurch gekennzeichnet, daß sie Ölsäure, Palmitoleinsäure oder cis-6-Hexadecensäure enthält.

4. Zubereitung gemäß Anspruch 1, dadurch gekennzeichnet, daß sie Wollwachsalkohole, Cholesterin, Cholesterinderivate und/oder Ceramide und Ölsäure enthält.

5. Zubereitung gemäß Anspruch 1, dadurch gekennzeichnet, daß sie 1 Gew.-% bis 5 Gew.-% Wollwachsalkohole, Cholesterin, Cholesterinderivate und/oder Ceramide und 0,2 Gew.-% bis 0,4 Gew.-% Ölsäure enthält.

6. Zubereitung gemäß Anspruch 1, dadurch gekennzeichnet, daß sie Antioxidantien enthält.

7. Kosmetische Verwendung der Zubereitungen gemäß Anspruch 1 als Hautpflegeprodukte, zur Pflege trockener Haut und von Altershaut.

8. Verwendung der Kombination aus Neutrallipid und mono-cis-Alkencarbonsäure gemäß Anspruch 1 zur Herstellung einer dermatologischen Zubereitung zur Behandlung und Prophylaxe von pathologisch trockener Haut, Neurodermitis und Folgeschäden der Hautaustrocknung, Fissuren, inflammatorischen oder allergischen Prozessen.

## Claims

1. Cosmetic and dermatological preparation containing at least one neutral lipid and at least one mono-cis-alkenecarboxylic acid or a salt thereof, the amount of mono-cis-alkenecarboxylic acid being 0.05 to 0.5 per cent by weight based on the total weight of the preparation.

2. Preparation according to Claim 1, characterized in that it contains, as neutral lipids, wool wax alcohols, cholesterol, cholesterol derivatives, ceramide, squalene, wax esters or triglycerides.

3. Preparation according to Claim 1, characterized in that it contains oleic acid, palmitoleic acid or cis-6-hexadecenoic acid.

4. Preparation according to Claim 1, characterized in that it contains wool wax alcohols, cholesterol, cholesterol derivatives and/or ceramides and oleic acid.

5. Preparation according to Claim 1, characterized in that it contains 1 % by weight to 5 % by weight of wool wax alcohols, cholesterol, cholesterol derivatives and/or ceramides and 0.2 % by weight to 0.4 % by weight of oleic acid.

6. Preparation according to Claim 1, characterized in that it contains antioxidants.

7. Cosmetic use of the preparations according to Claim 1 as skincare products for the care of dry skin and aged skin.

8. Use of the combination of neutral lipid and mono-cis-alkenecarboxylic acid according to Claim 1 for the production of a dermatological preparation for the treatment and prophylaxis of pathologically dry skin, neurodermitis and the damage as a result of the skin drying out, fissures, inflammatory or allergic processes.

## Revendications

1. Composition cosmétique et dermatologique, contenant au moins un lipide neutre et au moins un acide mono-*cis*-alcènecarboxylique ou un de ses sels, la proportion de l'acide mono-*cis*-alcènecarboxylique allant de 0,05 à 0,5 % en poids, par rapport au poids total de la composition.

2. Composition selon la revendication 1, caractérisée en ce qu'elle contient, en tant que lipides neutres, des alcools de lanoline, du cholestérol, des dérivés de cholestérol, un céramide, du squalène, des esters de cires ou des triglycérides.

3. Composition selon la revendication 1, caractérisée en ce qu'elle contient de l'acide oléique, palmitoléique ou *cis*-6-hexadécénoïque.

4. Composition selon la revendication 1, caractérisée en ce qu'elle contient des alcools de lanoline, du cholestérol, des dérivés de cholestérol et/ou des céramides et de l'acide oléique.

5. Composition selon la revendication 1, caractérisée en ce qu'elle contient 1 % en poids à 5 % en poids d'alcools de lanoline, de cholestérol, de dérivés de cholestérol et/ou de céramides, et 0,2 % en poids à 0,4 % en poids d'acide oléique.

6. Composition selon la revendication 1, caractérisée en ce qu'elle contient des antioxydants.

7. Utilisation cosmétique des compositions selon la revendication 1, en tant que produits de soins de la peau, pour le soin de la peau sèche et de la peau âgée.

8. Utilisation de l'association de lipide neutre et d'acide mono-*cis*-alcènecarboxylique selon la revendication 1, pour la préparation d'une composition dermatologique destinée au traitement et à la prophylaxie de la peau pathologiquement sèche, de la névrodermite et d'altérations faisant suite au dessèchement de la peau, de crevasses, de processus inflammatoires ou allergiques.
